# EUROPEAN PATENT SPECIFICATION

(11) **EP 1 418 891 B1**
(45) Date of publication and mention of the grant of the patent: **09.11.2005**
(21) Application number: 02755248.8
(22) Date of filing: 23.08.2002
(51) Int. Cl.: A61K 9/107, A61K 31/05, A61K 31/445, A61K 47/10, A61K 47/34

(54) **ANAESTHETIC FORMULATIONS OF PROPOFOL**
ANAESTHETISCHE PROPOFOL ZUBEREITUNGEN
FORMULATIONS ANESTHESIQUES DE PROPOFOL

(30) Priority: 24.08.2001 GB 0120702
(43) Date of publication of application: 19.05.2004
(73) Proprietor: Maelor Pharmaceuticals Limited, Newbridge, Wrexham LL14 3JG (GB)
(72) Inventor: MEADOWS, John Maelor Pharmaceuticals Limited, Newbridge Wrexham LL14 3JG (GB); HIGGINBOTTOM, Simon Maelor Pharmaceutical Limited, Newbridge Wrexham LL14 3JG (GB)
(74) Representative: Lord, Hilton David
(86) International application number: PCT/GB2002/003911
(87) International publication number: WO 2003/017977

(56) References cited:
- WO-A-00/78301
- WO-A-01/64187

## Description

The present invention relates to novel formulations of Propofol, as well as to their use and methods of manufacture.

Propofol [2,6-bis(1-methylethyl)phenol; 2,6-diisopropylphenol] is an injectable anaesthetic first described as far back as 1956. Early preparations of Propofol were formulated with polyethoxylated castor oil. Anaphylactoid reactions were observed with these formulations, and the currently favoured formulation is an oil-in-water preparation comprising soya bean oil and purified egg phosphatide (marketed as Diprivan by Zeneca). The formulation of Diprivan is as follows:

| **Substance** | **Amount** | **Role** |
|---|---|---|
| Propofol | 10.0mg | Active |
| Soya Bean Oil | 100.0mg | Oil |
| Egg Lecithin | 12.0mg | Emulsifier |
| Glycerol | 22.5mg | Osmotic Agent |
| Sodium Hydroxide | q.s. to pH8.5 | |
| Water | q.s. to 1.0ml | |

The presence of lecithin and soya bean oil makes Diprivan suitable as a growth medium for micro-organisms, so that care must be exercised to avoid contamination of the formulation after opening the vial.

Diprivan is associated with a high occurrence of severe pain on injection, and there have been many studies, both to minimise the pain, as well as to find alternative formulations. In general, the latter have focused on the preparation of emulsions, such as those using triglycerides, but these have tended to exhibit no particular advantage over Diprivan and suffer from similar problems, insofar as they must be prepared under strictly aseptic conditions.

Free Propofol is associated with pain on injection. In this respect, the term "free", as used herein, relates to Propofol associated with the aqueous phase of a formulation, such as microdroplets of Propofol suspended therein, or the small amount that is capable of solubilising in water. For this reason, it is not appropriate to incorporate miscible co-solvents in formulations of Propofol, as the increased amounts of dissolved Propofol may elevate pain and any other side-effects associated with free Propofol.

Studies to minimise pain on injection are reviewed by Tan, C. H., *et al*. [Anaesthesia (1998), **53**, 468-476], and good results were obtained with lignocaine mixed with the Propofol. However, the emulsion formulations of Propofol, such as Diprivan, are electrostatically stabilised colloidal systems and, in such systems, the addition of inert electrolytes, such as lignocaine hydrochloride, reduces the inter-particle electrostatic repulsions to such an extent that aggregation and coalescence can occur. Thus, if coadministration of Propofol and lignocaine is to be undertaken, then the two actives have to be mixed immediately prior to use, which is undesirable from a practical perspective.

Poloxamers, which are also sold by BASF as Pluronics (US) and Lutrols (Europe), and by ICI as Synperonics, have been used for the solubilisation of drugs in the past. The drugs on which the poloxamers were tested either were difficult to administer by normal means, owing to their insolubility in water, or were thought likely to benefit from targeting, owing to their toxicity, for example.

Poloxamers, in general, are non-toxic polymeric surfactants and are poly(a-oxyethylene-*b*-oxypropylene-*a*-oxyethylene) triblock copolymers. Their solubility in water is generally good, but the properties of the individual poloxamers vary substantially. The pharmaceutical acceptability of various poloxamers is well established, with P407 and P188, in particular, being approved for parenteral administration.

There have been problems with targeting and dispensing drugs using poloxamers. Munshi, *et al*., [Cancer Letters, 118 (1997), 13-19] found that it was not possible for the drug to act in a normal manner, unless ultrasound was used to disrupt the micelles. The use of ultrasound in surgical techniques is not only expensive, but undesirable.

Kabanov, *et al*., [Journal of Controlled Release, 22 (1992), 141-158] disclose a self-assembling supramacromolecular complex comprising drug, poloxamer and antibodies to try to target the drug contained within the thus-formed complex. Targeting the micelles by incorporating antibodies is not necessary for a general anaesthetic.

Rapoport, N., [Colloids and Surfaces B-Biointerfaces (1999) vol. 16, no. 1-4, 93-111] addresses Pluronic micelles as drug carriers. In particular, it notes that Pluronic micelles must be stabilised, and rules out the possibility of direct radical cross-linking of micelle cores, as this compromises drug loading capacity. A second route involves adding a small concentration of vegetable oil into dilute Pluronic solutions which, apparently, decreased micelle degradation on dilution. Introduction of any extraneous agents is undesirable in an anaesthetic formulation. The preferred route was to polymerise a temperature-responsive LCST hydrogel in the core of the Pluronic micelles.

WO 00/78301 discloses Propofol formulated with a poloxamer. These formulations are associated with pain on administration.

In co-pending PCT publication no. WO 01/64187 we disclose aqueous, micellar poloxamer preparations comprising Propofol which are stable at low concentrations.

There is a need for a formulation of Propofol that addresses the problem of pain on injection. It has now, surprisingly, been found that micellar preparations of poloxamers containing Propofol prepared with local anaesthetics remain stable and serve to reduce the pain on injection associated with injectable formulations of Propofol.

Accordingly, in a first aspect, there is provided an aqueous, micellar poloxamer preparation comprising Propofol and a local anaesthetic.

Small amounts of pharmaceutically acceptable compounds can be used to solubilise Propofol in amounts greater than previously practicable. Thus, in preferred embodiments, water forms the major part of the formulation, by far. Further, such preparations have virtually no free Propofol, which serves to reduce pain on injection. What is more, the formulations of the present invention do not appear to substantially de-micellise, even at infinite dilutions, a property which is particularly valuable for injectibles, which are effectively infinitely diluted in the blood stream.

It is a particular advantage of the present invention, therefore, that pain on injection is already initially reduced by the solubilising effect of the poloxamer, but is further reduced by the incorporation of a local anaesthetic. Thus, pain is substantially reduced, and can often be reduced to nothing more than the pain associated with the injection apparatus itself.

The formulations of the present invention have the advantage of mixing Propofol with lignocaine, or other local anaesthetic, without having to prepare the injection immediately prior to use. These formulations cause a significantly reduced level of pain on injection compared to Diprivan.

Essentially, the formulations of the present invention have the advantage over Diprivan, and all other generic emulsions, in that, being non-ionic, the colloidal stability of the poloxamer micelles is unaffected by the increase in ionic strength of the aqueous phase associated with the inclusion of appropriate amounts of local anaesthetics, such as lignocaine hydrochloride.

The formulations of the present invention may incorporate any ionically dissociable water soluble local anaesthetic suitable for parenteral use. A particularly preferred example is lignocaine hydrochloride, but other anaesthetics are useful in the present invention, such as procaine hydrochloride, prilocaine hydrochloride, chloroprocaine hydrochloride, etidocaine hydrochloride, or mepivacaine hydrochloride.

Suitable levels of local anaesthetic for use in the present invention range from 0.01% to 1% and, more preferably, between 0.05% w/v to 0.40% w/v.

Poloxamers are surfactants, and surfactants are amphiphilic substances. In other words, they comprise both hydrophilic and hydrophobic regions, and are commonly used to solubilise fatty substances in water. Above certain concentrations in water, surfactants tend to form micelles - agglomerations of surfactant molecules presenting their hydrophilic portions to water and internalising the hydrophobic portions. With increasing concentration, other structures may also be observed, but these tend to be somewhat complex. In the obverse, each surfactant has a minimum concentration in water below which micelles disperse (critical micelle concentration - CMC), and the aqueous surfactant preparation is effectively a solution of unimers with no structure.

Surfactant micelles are effectively envelopes and, in water, will have the more hydrophobic portion of the molecule generally forming the inside of the envelope. These micelles can readily interact with other substances and, if the substance is an oil, for example, then the substance can be entirely internalised within the micelle, or otherwise form an association, thereby effectively solubilising the substance in water.

It is undesirable for Propofol to be released as the free oil into the bloodstream, for the reasons noted above. Accordingly, using a surfactant system to solubilise Propofol in an aqueous preparation would be expected to present an unacceptable risk, with any surfactant micelles liable to disperse at lower concentrations, especially at infinite dilution, such as would be encountered on injection.

Instead, however, it has been discovered that Propofol actually encourages micelle formation of the poloxamers in water, at temperatures and concentrations lower than would otherwise be expected, and that, once the micelles contain Propofol, they remain stable at infinite dilution. Although not essential to the present invention, it is believed that Propofol is internalised within the micelle and serves to dramatically enhance the stability of the micelle. Furthermore, *in vivo* tests (see *in vivo* Test Example below) have demonstrated that the aqueous Propofol preparations of the invention are at least as effective as Diprivan, and that they show none of the side effects that would be noted if the micelles disaggregated on dilution in the bloodstream.

The nature of the poloxamer is not essential to the present invention although, especially where the formulation is intended for administration to a human, it should be pharmaceutically acceptable.

Despite the findings in the art, it has surprisingly been found that Propofol, alone, is not only sufficient to stabilise the poloxamer micelles, but that there is no requirement for the micelles to be targeted and that an extremely simple mix of Propofol, surfactant and water is sufficient to make up an anaesthetic, or sedative, formulation of the invention. Furthermore, the mix may be autoclaved without problem, and may generally be prepared by simple roller mixing, as the preparations are thermodynamically stable, and readily form.

Poloxamers are generally unreactive and non-responsive to any other additives to the system, such as BSA (Bovine Serum Albumin) or salt, such as sodium chloride. In addition, pH appears to have little, or no, effect. Thus, there is no problem with incorporating suitable substances to render the Propofol formulation suitable for injection. In particular, it is preferred that the Propofol formulation of the invention should be isotonic with the blood, so as not to cause any haemolysis, for example. Other suitable additives include chelating agents, such as EDTA, and antioxidants, which can assist in preventing discoloration of the Prpofol.

Poloxamers vary greatly in their constituent make up, and are generally characterised by the ratio of ethylene oxide (EO) units to propylene oxide units, and the molecular weight of the propylene oxide (PO) block. Within the general range of poloxamers available, it has generally been found that those having an average molecular weight of propylene oxide of greater than 1500 D and an average percent ethylene oxide of greater than 30% w/w are suitable. More preferably, the PO portion is at least 2000 D while the EO portion is at least 40% w/w. However, where mixtures of poloxamers are employed, this general rule does not apply.

Where preparations of the present invention comprise a single poloxamer, then these preferably contain at least 0.8% w/w Propofol, with formulations containing 1% w/w being more preferred. For veterinary use, formulations comprising lower concentrations, such as 0.3 to 0.7%, preferably 0.5%, may also be useful. The upper end of the range is generally dictated by the ability of the system to support higher concentrations of Propofol. With concentrations of 10% w/w poloxamer in water, the maximum concentration of Propofol is about 3.2% when a poloxamer such as P237 is used. Poloxamer combinations can take this even higher. However, a physiologically effective concentration is 1%, so that higher concentrations result in smaller volumes being required which can be awkward to administer. Thus, a Propofol concentration in the range of 1% - 1.5% w/w is preferred.

Individually preferred poloxamers are P188 , P234, P237, P338 and P407. P407 and P188 are particularly preferred as they have been approved for medicinal purposes. P234 and P338 are generally better than P407, but neither has been approved. Likewise, P237 provides excellent uptake, but also has yet to be approved.

Advantageously, combinations of poloxamers are employed in the present invention. Surprisingly, it has been found that such combinations are synergistic, where the PO blocks have different sizes. Without being bound by theory, this is thought to be because of the formation of mixed micelles.

As noted above, poloxamers comprise PO units and EO units. The PO units are generally hydrophobic, and form the central portion of any micelle. In micelles with only one poloxamer, PO blocks align with each other, while EO blocks also align with each other on the outside, to form a thermodynamically stable system. In a mixed micelle, with poloxamers of differing PO length, when the PO blocks of different poloxamers align, either a "hole" is left in the micellar interior, or part of the EO block of the shorter poloxamer must align with the PO of the larger molecule. This is not thermodynamically stable and, with poloxamers that are substantially different, happens virtually not at all.

With Propofol present, these problems are overcome, and the Propofol actually encourages the formation of mixed micelles. It would appear that the Propofol compensates for the difference in PO length, by occupying the space at the end of the shorter PO chain, thereby obviating the need for either a thermodynamically unfavourable association of EO and PO, or any tendency toward "holes", or both.

In practice, it will be appreciated that actual 'holes' would not be present within the micellar interior, as neighbouring PO blocks would adjust their configurations accordingly to compensate for the 'mismatch' in chain lengths. However, such configurational requirements would be entropically restrictive and, thus, thermodynamically unfavourable.

This ability of Propofol to stabilise mixed micelles has numerous advantages. First, it stabilises the micelle to the extent that the micelle does not disaggregate even at infinite dilution, once formed, so that no free Propofol is released by the effects of dilution alone. Second, the effect is sufficiently strong, that poloxamers which do not normally micellise, or are otherwise only sparingly soluble in water, readily form micelles in the presence of Propofol and another poloxamer, and vigorous mixing simply is not usually necessary. Third, the micelles are thermodynamically stable, so that they will not disaggregate on storage and, if heated to disruption, will simply reform on cooling. Fourth, synergistically formed, mixed micelles effectively actively trap Propofol, so that even less free Propofol is available in aqueous solution, thereby further reducing pain on injection. Finally, in synergistic mixtures, less poloxamer is required to solubilise 1% Propofol or, concomitantly, the same amount ensures that substantially all free Propofol is mopped up.

For example, the poloxamer known as P407 (also known as F127) has synergistic properties with P 188 (also known as F68), such that the maximum concentration of Propofol able to be solubilised in a 10% w/v aqueous solution of poloxamer is at its greatest when the ratio of P407 to P 188 is 7 : 3 by weight. This is particularly surprising, given that a 10% w/v solution of P188 in water can only support a maximum Propofol concentration of 0.8%, and a 10% w/v solution of P407 can support a maximum concentration of Propofol of 1.7%, whereas the 7 : 3 ratio of the two poloxamers can support a maximum concentration of Propofol of 3.5 - 3.8%.

Thus, in a preferred embodiment, the present invention provides an aqueous preparation of Propofol wherein the Propofol is solubilised in a synergistic mix of poloxamers.

There are preferably only two poloxamers.

The preferred concentrations of Propofol are as defined above.

As noted above, the PO blocks of synergistic poloxamers appear to be of different weights, although it is readily determined by one skilled in the art as to which combinations of poloxamers are synergistic. Even a combination of P108 and P188 is synergistic, although P108 solubilises less than 0.1 % Propofol, on its own (10% P 108 in water), and may be used advantageously with P188, for example.

It appears that P401 has too little EO, and is not particularly useful in the present invention, as its lack of solubility in water is not significantly overcome by Propofol.

In general, provided that there is a difference between two poloxamers, particularly between the PO portions, then a synergistic mixture will form. For example, a mix of P237 and P234, or a mix of P188 and P184, is not synergistic, but other mixes, such as: P407 with P338, P234, P237, P188 or P108; P338 with P234, P237, P188 or P108; P234 with P188 or P108; or P237 with P188 or P108 are all useful.

Any synergistic ratio is acceptable and useful. In general, a ratio of from 1 : 1 to 8 : 2 w/w is useful, with 1 : 1 to 7 : 3 being preferred.

It will be appreciated that the present invention further provides a method for the anaesthesia of a mammalian, preferably human, patient by the administration of an effective amount of a preparation of the present invention thereto.

The hydrodynamic radii of micelles of poloxamers tend not to exceed about 10 - 20 nm, and are readily filterable through a 0.2 µm filter. Such filters are used commercially in order to sterilise formulations, and this is a further advantage of the present invention. A major drawback with Diprivan is the lack of options for sterilisation of the formulation. It cannot be filtered through a 0.2 µm filter, as the size of the emulsion particles is generally in the region of 300 nm (0.3 µm), and the emulsion is also too unstable to be autoclaved. By contrast, the formulations of the present invention are thermodynamically stable so that they can be both filtered to sterility and/or autoclaved.

Autoclaving may be undesirable where filtering has achieved the required effect, and it should also be noted that autoclaving can have the effect of disrupting the micelles and the formulation in general, to the extent that re-mixing of the formulation may be required after autoclaving. This generally poses no particular problem because the formulations of the present invention are thermodynamically stable and, therefore, the constituents readily return to the favoured state of the formulation, although it can be inconvenient. It should also be noted that autoclaving may not be suitable if other constituents are present in the sterile formulation and which may be adversely affected by elevated temperatures.

Preparation of formulations of the present invention is generally straightforward. Although the constituents of the formulations can be added in any sequence, as desired, it will be appreciated that Propofol is virtually insoluble in water, so that the generally commercially desirable method of mixing is to prepare a poloxamer solution in water, followed by the addition of Propofol.

P407 is readily soluble in water, but heating of the water and the poloxamer, whilst mixing, can generally increase the speed of micelle formation. In addition, some poloxamers require increased temperatures in order to satisfactorily micellise in water. In general, concentrations of poloxamer of 10% w/v are useful in the present invention, but concentrations of poloxamers, whether single or mixed, can be selected by those skilled in the art, and will generally be above 0.5% and below 20%. More preferred concentrations are from 3 to 12%. Some poloxamers will begin to gel at higher concentrations, and any poloxamer concentration that gels at body temperature, especially when in association with Propofol, should be avoided for injection purposes. Preferred poloxamer mixes are those that enhance Propofol uptake and/or inhibit gelling, particularly at body temperature.

A formulation of 9% P407 / 4% p188 works well to solubilise 1% Propofol, although the viscosity of such a preparation is about 6 times greater than that of Diprivan. Retaining the relevant ratio, a formulation containing 3% P407 / 1.33% P188 comfortably solubilises 1% Propofol and displays essentially identical features to the higher strength poloxamer formulation, including micellar size and stability to dilution, while exhibiting a similar viscosity to that of Diprivan.

Propofol can be added at any stage, but it is currently preferred to add Propofol to an aqueous solution of the poloxamer. Propofol is naturally an oil, and can simply be added to the poloxamer solution and incorporated into the solution in a roller mixing technique. Likewise, the anaesthetic can be added to the poloxamer solution, together with Propofol, or before or after Propofol. However, as the anaesthetic is generally readily water soluble, it may be added to the water phase together with the poloxamer component, or even before, if desired.

The hydrodynamic size of the micelles containing Propofol does not appear to be dependent on the nature of the mixing process involved. Gentle roller mixing achieves solubilisation of the Propofol slightly more slowly than high shear mixing, but high shear mixing tends to result in foaming, and the resulting head needs to be allowed to settle before the solution can be used.

Solubilisation of Propofol into aqueous poloxamer solution occurs spontaneously upon gentle agitation. The fact that stable homogeneous systems can be prepared using such gentle agitation is indicative that the incorporation of Propofol into the aqueous system is through a mechanism of solubilisation into poloxamer micelles rather than through any emulsification mechanism. Micellar solubilisation, such as the incorporation of Propofol into P407 micelles, results in a thermodynamically stable system. It is energetically favourable for this type of system to form, so only gentle agitation is required to facilitate adequate contact between the solubilising vehicle and the solubilised species. In contrast, most emulsions can be classed as kinetically stable systems. With such systems, sufficient energy must be applied to overcome a significant activation energy before they can form; this energy is usually applied through some form of high shear mixing. Similarly, once formed, there is a significant activation energy barrier to any de-emulsification process, although these systems may be broken, given sufficient time or the input of sufficient energy, e.g. centrifugation. Although kinetically stable systems can remain stable for a long time, thermodynamically stable systems, such as those of the present invention, have, technically, infinite long-term stability.

Diprivan has a Propofol concentration of about 1% w/v, and this appears to work well. Concentrations of Propofol in the formulations of the present invention are preferably formulated to contain an amount of Propofol to be about the equivalent of the Diprivan formulation, and it has been established that formulations of the present invention containing 1% Propofol have similar pharmacological properties to Diprivan. Lower concentrations of Propofol require the administration of concomitantly greater volumes of the formulation of the invention, while higher concentrations need to be handled with greater care. Thus, Propofol concentrations in the range of 0.5 to 2% are generally preferred, with 1% being most preferred.

Formulations of the present invention are easily prepared at a fraction of the cost of the manufacture of Diprivan; they can be sterilised after preparation; they have no constituents which encourage the multiplication of micro-organisms in the formulation; they are substantially stable, and they are associated with considerably less pain on injection, all of which properties are in direct contrast to Diprivan.

The formulations of the present invention need very few constituents. Propofol, surfactant and water is sufficient for a basic formulation, but it is greatly preferred that any injectable formulation is made up with saline, for example, in order to render the formulation isotonic, or iso-osmotic, with blood. In the preparations of the present invention, an appropriate level is 0.4 to 0.6%, more preferably 0.45%, in order to achieve an osmolality of 300 mOsm, with a range of 280 - 320 mOsm being generally desirable. Anything outside of this range may be used, but may possibly lead to perceptible pain.

Apart from the desirability of rendering the formulation isotonic with blood, it is generally preferred to minimise the number of other ingredients and to ensure that any formulation passed on to the patient is sterile. Given that the formulation can be sterilised after preparation and that simple preparations of the invention do not readily support growth of micro-organisms, then this is not a problem. Nevertheless, if it is desired to incorporate sterilising agents, stabilising agents, or bacteriostats, for example, then this can be done, and prior art formulations of Propofol have included sodium metabisulphite and EDTA (ethylene diamine tetraacetic acid), which may be incorporated in the formulations of the present invention, if desired. It will be appreciated that other additives may also be employed, such as antioxidants.

It will be readily appreciated by those skilled in the art how to administer formulations of the present invention to a human or animal. Less Propofol is generally required with increasing age but, in general, there appears to be no particular effect of sex or body mass on the overall requirement of Propofol, and that amounts of Propofol in the region of 1.5 mg/kg to 2.5 mg/kg is generally sufficient for the induction of general anaesthesia, whilst long term infusion for anaesthesia requires a dose of 4 - 12 mg/kg per hour, the maximum effect being within one minute of dosing and duration of action being 5 to 10 minutes after administration. Lower consistent doses can provide sedation. The formulations of the present invention are generally intended for administration to the patient by parenteral injection, but other forms of administration, such as *via* a catheter, provide a similar effect. In general, administration into a relatively large vein is preferred, in order to minimise any pain.

The formulations of the present invention can be provided in any suitable form and may be provided in any suitable containers appropriate to maintaining sterility. If necessary, the containers may be autoclaved immediately prior to use, although this is not preferred, and is not generally convenient.

The formulations of the present invention may also be provided as concentrates, although high concentrations of surfactants are generally not preferred and, in the case of certain poloxamers, can lead to gelation which is undesirable. Accordingly, it is generally preferred that the formulations of the present invention are provided in a form suitable for direct injection. In such a capacity, any ampoule (for example) containing the formulation of the invention may, as appropriate, be used directly in a suitably adapted syringe to administer the formulation.

More generally, the ampoule, or other container, may be pierceable, or have a removable seal or cap, such that a syringe may be used to extract the solution, or the solution may be pourable directly into a syringe, or other apparatus for dosing the patient.

The present invention will now be illustrated with respect to the following, nonlimiting Examples in which, unless otherwise stated, all percentages are weight by volume and water used is sterile, deionised water.

### EXAMPLE 1

### Preparation of Samples

### Poloxamer Stock Solutions (500 ml):

10 % w /v poloxamer solutions were prepared by adding 50 g of poloxamer, or poloxamer mix, to 350 ml of distilled water. This was then mixed using an overhead stirrer until completely dissolved. This solution was then made up to 500 ml with distilled water.

### Propofol Formulations (20 ml):

1% w/w Propofol formulations were prepared by adding 0.2 g of Propofol to 20 ml of a stock solution, as prepared above. The solutions were then placed on a roller mixer to mix until all the Propofol had been solubilised (determined by visual evaluation), usually overnight, or for a sufficiently long period of time, sometimes up to 72 hours.

### EXAMPLE 2

### Maximum Propofol Concentrations in Poloxamer Solutions

In order to determine the maximum additive concentration (MAC) of Propofol in surfactant systems, the appropriate amount of Propofol was added to 30 ml of the stock solution and mixed as described in Example 1 until Propofol was solubilised, or until it was apparent that that amount of Propofol could not be solubilised. Ten ml aliquots were taken from samples in which Propofol had been completely solubilised, for particle size analysis. The concentration of Propofol in the remaining solution was increased by adding the appropriate amount of Propofol and mixing as above. This process was repeated for each poloxamer / surfactant solution until the maximum additive concentration (MAC) of Propofol was determined.

Table 1 below shows the results for the determination of the maximum additive concentration (MAC) for Propofol in the poloxamer solutions studied.

**Table 1**

| MAC for Propofol in a Number of Poloxamer solutions at 10% w/v at 25°C. | | | | |
|---|---|---|---|---|
| Poloxamer | Composition | % w/w PO | % w/w PEO | MAC* (% w/v) |
| Synperonic PE - L44 (P124) | PO 21Units, EO 14Units | 60 | 40 | <0.1% |
| Lutrol F 68 (P188) | PO 30Units, EO 120Units | 20 | 80 | 0.8% |
| Lutrol F 87 (P237) | PO 39Units, EO 156Units | 30 | 70 | 3.2 - 3.3% |
| Lutrol F 108 (P338) | PO 56Units, EO 224Units | 20 | 80 | 2.0-2.2% |
| Lutrol F 127 (P407) | PO 57Units, EO 196Units | 30 | 70 | 1.5-1.7% |

| | | | | |
|---|---|---|---|---|
| *MAC: the lowest figure represents the highest amount of Propofol deemed to be fully solubilised i.e. optically clear; the higher figure represents the lowest concentration of Propofol deemed not to be fully solubilised | | | | |

There appears to be no obvious correlation between poloxamer structure in terms of %PO and PO block length, and MAC. Studies of 5% w/v poloxamer solutions have shown that P124 and P188 do not micellise until 40°C and 57°C respectively and, so, are not present as micelles in aqueous solution at room temperature. However, the above results indicate that the presence of Propofol in P188 systems induces micelle formation at room temperature. Similarly, where P237 at 5% w/v has been shown to micellise at around 34°C, the presence of Propofol appears to induce micellisation at room temperature, hence enabling it to solubilise large amounts of Propofol.

Table 1a shows the results of a separate set of experiments (MAC of Propofol in 10% w/w aqueous solutions of various poloxamers with simple roller mixing at room temperature).

**Table 1a**

| **Poloxamer** | **MAC (% w/w)** |
|---|---|
| Lutrol F 87 (P237) | 3.2 |
| Lutrol F 108 (P338) | 2.2 |
| Lutrol F 127 (P407) | 1.7 |
| Lutrol F 68 (P188) | 0.8 |
| Synperonic PE - F84 (P234) | 2.0 |
| Synperonic PE - L121 (P401) | 0.1 |
| Synperonic PE - L 64 (P184) | 0.1 |
| Synperonic PE - L 44 (P124) | <0.1 |
| Synperonic PE - F 38 (P108) | <0.1 |

The nomenclature used for the "P" poloxamers in this Example, and generally herein, is such that the first two figures, when multiplied by 100, represent the average molecular weight of the PO block, whilst the last figure, when multiplied by 10, represents the ethylene oxide content (% w/w) of the poloxamer. Thus, for P407, the average molecular weight of the PO block is 4000 Daltons with a 70% w/w/ ethylene oxide content.

It can be seen that, with a PO of less than 1800 D, or an EO of less than 40%, then a 10% w/w aqueous solution of the poloxamer essentially becomes incapable of supporting a solution of 0.8% Propofol.

### EXAMPLE 3

### Solubility of Propofol in Water

Analyses were performed using the Perkin Elmer Lambda 5 UV/Vis Spectrophotometer.

Two sets of standards were prepared by serial dilution of:
1. 10% poloxamer P407/1% Propofol solution in water.
2. 1% Propofol solution in ethanol (EtOH).
   Dilutions were performed with water and EtOH respectively. The standards were further diluted one hundred fold prior to measurement of their UV absorption spectra recorded.

The wavelength of maximum absorption (λₘₐₓ) for Propofol is around 272 nm and was unchanged for a 1% solution in ethanol, a 1% solution in 10% P407 in water, or a saturated aqueous solution. Graphs for both the ethanol and poloxamer solutions are linear up to Propofol concentrations of 0.02% w/v. Using these, it was possible to estimate the concentration of Propofol in a saturated aqueous solution as approximately 1 × 10⁻³ M.

### EXAMPLE 4

### Mixed Poloxamer Systems

The MAC values of Propofol in various mixed poloxamer systems, at a total poloxamer concentration of 10% w/w, are shown in Table 2, below. All values are in % w/w.

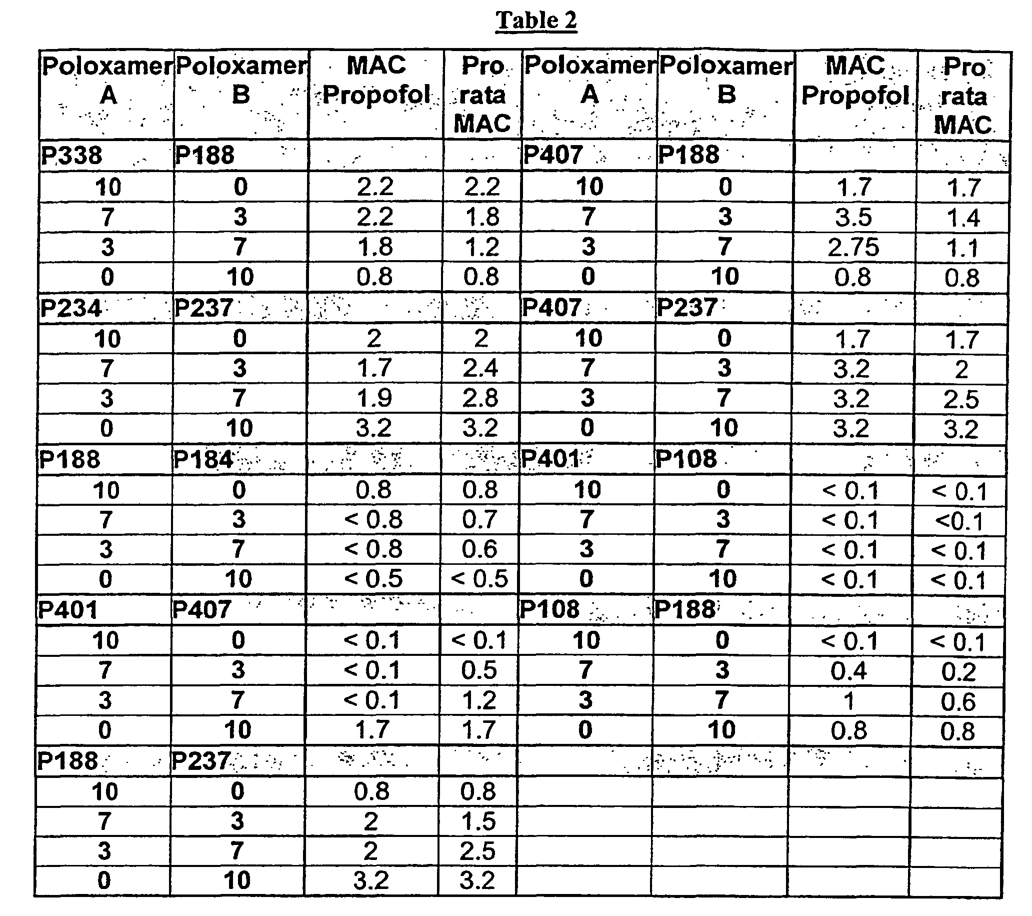

Synergy is established when the experimentally determined MAC for the mixed systems is greater than the value calculated from the pro rata addition of the solubilisation capacity of each of the Poloxamer components individually. These pro rata values are included in the table for comparison.
Example calculation : P407 / P188 mixtures
MAC 10% P 407 = 1.7% MAC 10% P 188 = 0.8%
Pro rata MAC for 7% P 407 / 3% P 188 = (0.7 x 1.7) + (0.3 x 0.8)
= 1.19+0.24
= 1.4%
Similarly for 3% P407 / 7% P 188, Pro rata MAC = 1.1%

Accordingly, synergy in the above Table is demonstrated by the mixtures: P 407/P188; P407/P237; P338/P188 and P188/P108. The dissimilarity in the PO block lengths of the mixtures is notable.

The mixtures: P234 / P237; P188 / P184; P401 / P407; and P 401 / P108 displayed no evidence of synergy. The similar PO block lengths of the mixtures is notable, except for P401/P108. In this case, P401 is essentially insoluble in water at room temperature, and this does not appear to be counteracted by P108.

### EXAMPLE 5

### Formulation

An anaesthetic formulation was prepared, as follows:
3% w/v Poloxamer 407;
1.33% w/v Poloxamer 188;
1% w/v Propofol;
0.1% w/v lignocaine hydrochloride;
sodium chloride (as required to render solution isotonic).

All components except the Propofol were dissolved in the water. The Propofol was then added with gentle mixing until completely solubilised.

In each of the following Examples 6 to 39, three formulations are prepared as described, each with a different tonicity modifying agent. The tonicity modifying agents used are glucose, sodium lactate and sodium chloride, and are added, as required, to render the formulation iso-osmotic. In addition to the listed components, the pH of the formulation was adjusted, as required, to pH 6.0 - 7.0 with either dilute aqueous sodium hydroxide solution or dilute hydrochloric acid solution. The formulations were prepared by dissolving all of the components, except the Propofol, in water for injections, which was subsequently added, *q*.*s*., to 100 ml. The Propofol was then added, with gentle mixing, until completely solubilised.

### EXAMPLE 6

The three anaesthetic formulations are prepared as described, with the following components:
9 % w/v Poloxamer 407;
4% w/v Poloxamer 188;
1% w/v Propofol;
0.1% w/v Lignocaine hydrochloride; and
0.00005% w/v Edetic acid (ethylenediamine tetraacetic acid, EDTA).

### EXAMPLE 7

The three anaesthetic formulations are prepared as described, with the following components:
8.3% w/v Poloxamer 407;
3.7 % w/v Poloxamer 188;
1% w/v Propofol;
0.1 % w/v Lignocaine hydrochloride; and
0.00005% w/v Edetic acid.

### EXAMPLE 8

The three anaesthetic formulations are prepared as described, with the following components:
7.6% w/v Poloxamer 407;
3.4 % w/v Poloxamer 188;
1% w/v Propofol;
0.1 % w/v Lignocaine hydrochloride; and
0.00005% w/v Edetic acid.

### EXAMPLE 9

The three anaesthetic formulations are prepared as described, with the following components:
6.9% w/v Poloxamer 407;
3.1% w/v Poloxamer 188;
1% w/v Propofol;
0.1% w/v Lignocaine hydrochloride; and
0.00005% w/v Edetic acid.

### EXAMPLE 10

The three anaesthetic formulations are prepared as described, with the following components:
6.2% w/v Poloxamer 407;
2.8% w/v Poloxamer 188;
1% w/v Propofol;
0.1% w/v Lignocaine hydrochloride; and
0.00005% w/v Edetic acid.

### EXAMPLE 11

The three anaesthetic formulations are prepared as described, with the following components:
7.1 % w/v Poloxamer 407;
5.9 % w/v Poloxamer 188;
1 % w/v Propofol;
0.1% w/v Lignocaine hydrochloride; and
0.00005% w/v Edetic acid.

### EXAMPLE 12

The three anaesthetic formulations are prepared as described, with the following components:
6.6% w/v Poloxamer 407;
5.4 % w/v Poloxamer 188;
1% w/v Propofol;
0.1 % w/v Lignocaine hydrochloride; and
0.00005% w/v Edetic acid.

### EXAMPLE 13

The three anaesthetic formulations are prepared as described, with the following components:
6 % w/v Poloxamer 407;
5% w/v Poloxamer 188;
1% w/v Propofol;
0.1 % w/v Lignocaine hydrochloride; and
0.00005% w/v Edetic acid.

### EXAMPLE 14

The three anaesthetic formulations are prepared as described, with the following components:
5 % w/v Poloxamer 407;
5% w/v Poloxamer 188;
1% w/v Propofol;
0.1% w/v Lignocaine hydrochloride; and
0.00005% w/v Edetic acid.

### EXAMPLE 15

The three anaesthetic formulations are prepared as described, with the following components:
4 % w/v Poloxamer 407;
6% w/v Poloxamer 188;
1% w/v Propofol;
0.1% w/v Lignocaine hydrochloride; and
0.00005% w/v Edetic acid.

### EXAMPLE 16

The three anaesthetic formulations are prepared as described, with the following components:
9 % w/v Poloxamer 407;
4% w/v Poloxamer 188;
1% w/v Propofol;
0.1% w/v Lignocaine hydrochloride;
0.17% w/v Ketamine hydrochloride; and
0.00005% w/v Edetic acid.

### EXAMPLE 17

The three anaesthetic formulations are prepared as described, with the following components:
6.6 % w/v Poloxamer 407;
5.4% w/v Poloxamer 188;
1% w/v Propofol;
0.1% w/v Lignocaine hydrochloride;
0.17% w/v Ketamine hydrochloride; and
0.00005% w/v Edetic acid.

### EXAMPLE 18

The three anaesthetic formulations are prepared as described, with the following components:
6 % w/v Poloxamer 407;
5 % w/v Poloxamer 188;
1% w/v Propofol;
0.1 % w/v Lignocaine hydrochloride;
0.17% w/v Ketamine hydrochloride; and
0.00005% w/v Edetic acid.

### EXAMPLE 19

The three anaesthetic formulations are prepared as described, with the following components:
5 % w/v Poloxamer 407;
5 % w/v Poloxamer 188;
1% w/v Propofol;
0.1% w/v Lignocaine hydrochloride;
0.17% w/v Ketamine hydrochloride; and
0.00005% w/v Edetic acid.

### EXAMPLE 20

The three anaesthetic formulations are prepared as described, with the following components:
5 % w/v Poloxamer 407;
5 % w/v Poloxamer 188;
1 % w/v Propofol;
0.1 % w/v Lignocaine hydrochloride;
0.01 % Alfentonil hydrochloride; and
0.00005% w/v Edetic acid.

### EXAMPLE 21

The three anaesthetic formulations are prepared as described, with the following components:
6.6 % w/v Poloxamer 407;
5.4 % w/v Poloxamer 188;
1% w/v Propofol;
0.1 % w/v Lignocaine hydrochloride;
0.01 % Alfentonil hydrochloride; and
0.00005% w/v Edetic acid.

### EXAMPLE 22

The three anaesthetic formulations are prepared as described, with the following components:
9 % w/v Poloxamer 407;
4 % w/v Poloxamer 188;
1 % w/v Propofol;
0.1% w/v Lignocaine hydrochloride;
0.01 % Alfentonil hydrochloride; and
0.00005% w/v Edetic acid.

### EXAMPLE 23

The three anaesthetic formulations are prepared as described, with the following components:
9 % w/v Poloxamer 407;
4% w/v Poloxamer 188;
1% w/v Propofol;
0.1 % w/v Chloroprocaine hydrochloride; and
0.00005% w/v Edetic acid.

### EXAMPLE 24

The three anaesthetic formulations are prepared as described, with the following components:
6.6 % w/v Poloxamer 407;
5.4 % w/v Poloxamer 188;
1 % w/v Propofol;
0.1 % w/v Chloroprocaine hydrochloride; and
0.00005% w/v Edetic acid.

### EXAMPLE 25

The three anaesthetic formulations are prepared as described, with the following components:
9 % w/v Poloxamer 407;
4% w/v Poloxamer 188;
1% w/v Propofol;
0.2% w/v Chloroprocaine hydrochloride; and
0.00005% w/v Edetic acid.

### EXAMPLE 26

The three anaesthetic formulations are prepared as described, with the following components:
9 % w/v Poloxamer 407;
4% w/v Poloxamer 188;
1% w/v Propofol;
0.1% w/v Ropivocaine hydrochloride; and
0.00005% w/v Edetic acid.

### EXAMPLE 27

The three anaesthetic formulations are prepared as described, with the following components:
6.6 % w/v Poloxamer 407;
5.4 % w/v Poloxamer 188;
1% w/v Propofol;
0.1% w/v Ropivocaine hydrochloride; and
0.00005% w/v Edetic acid.

### EXAMPLE 28

The three anaesthetic formulations are prepared as described, with the following components:
6.6 % w/v Poloxamer 407;
5.4 % w/v Poloxamer 188;
1% w/v Propofol;
0.1 % w/v Procaine hydrochloride; and
0.00005% w/v Edetic acid.

### EXAMPLE 29

The three anaesthetic formulations are prepared as described, with the following components:
9 % w/v Poloxamer 407;
4 % w/v Poloxamer 188;
1% w/v Propofol;
0.1% w/v Procaine hydrochloride; and
0.00005% w/v Edetic acid.

### EXAMPLE 30

The three anaesthetic formulations are prepared as described, with the following components:
9 % w/v Poloxamer 407;
4 % w/v Poloxamer 188;
1% w/v Propofol;
0.1 % w/v Prilocaine hydrochloride; and
0.00005% w/v Edetic acid.

### EXAMPLE 31

The three anaesthetic formulations are prepared as described, with the following components:
6.6 % w/v Poloxamer 407;
5.4 % w/v Poloxamer 188;
1% w/v Propofol;
0.1 % w/v Prilocaine hydrochloride; and
0.00005% w/v Edetic acid.

### EXAMPLE 32

The three anaesthetic formulations are prepared as described, with the following components:
9 % w/v Poloxamer 407;
4 % w/v Poloxamer 188;
1% w/v Propofol;
0.1% w/v Mepivacaine hydrochloride; and
0.00005% w/v Edetic acid.

### EXAMPLE 33

The three anaesthetic formulations are prepared as described, with the following components:
5 % w/v Poloxamer 407;
5 % w/v Poloxamer 188;
1% w/v Propofol;
0.1% w/v Mepivacaine hydrochloride; and
0.00005% w/v Edetic acid.

### EXAMPLE 34

The three anaesthetic formulations are prepared as described, with the following components:
6.6 % w/v Poloxamer 407;
5.4 % w/v Poloxamer 188;
1% w/v Propofol;
0.1% w/v Mepivacaine hydrochloride; and
0.00005% w/v Edetic acid.

### EXAMPLE 35

The three anaesthetic formulations are prepared as described, with the following components:
9 % w/v Poloxamer 407;
4% w/v Poloxamer 188;
1% w/v Propofol; and
0.1% w/v Lignocaine hydrochloride.

### EXAMPLE 36

The three anaesthetic formulations are prepared as described, with the following components:
7.6% w/v Poloxamer 407;
3.4 % w/v Poloxamer 188;
1 % w/v Propofol; and
0.1 % w/v Lignocaine hydrochloride.

### EXAMPLE 37

The three anaesthetic formulations are prepared as described, with the following components:
6.6% w/v Poloxamer 407;
5.4 % w/v Poloxamer 188;
1 % w/v Propofol; and
0.1% w/v Lignocaine hydrochloride.

### EXAMPLE 38

The three anaesthetic formulations are prepared as described, with the following components:
5 % w/v Poloxamer 407;
5% w/v Poloxamer 188;
1 % w/v Propofol; and
0.1% w/v Lignocaine hydrochloride.

### EXAMPLE 39

The three anaesthetic formulations are prepared as described, with the following components:
4 % w/v Poloxamer 407;
6% w/v Poloxamer 188;
1 % w/v Propofol; and
0.1 % w/v Lignocaine hydrochloride.

### EXAMPLE 40

The three anaesthetic formulations are prepared as described, with the following components:
10 % w/v Poloxamer 237;
1 % w/v Propofol;
0.1 % w/v Lignocaine hydrochloride; and
0.00005% w/v Edetic acid (ethylenediamine tetraacetic acid, EDTA).

### EXAMPLE 41

The three anaesthetic formulations are prepared as described, with the following components:
10 % w/v Poloxamer 338;
1 % w/v Propofol;
0.1% w/v Lignocaine hydrochloride; and
0.00005% w/v Edetic acid.

### EXAMPLE 42

The three anaesthetic formulations are prepared as described, with the following components:
7 % w/v Poloxamer 237;
3% w/v Poloxamer 234;
1% w/v Propofol;
0.1 % w/v Lignocaine hydrochloride; and
0.00005% w/v Edetic acid.

## Claims

1. An aqueous, micellar poloxamer preparation comprising Propofol and a local anaesthetic.

2. A preparation according to claim 1, wherein the anaesthetic is an ionically dissociable, water soluble, local anaesthetic.

3. A preparation according to claim 1 or 2, wherein the anaesthetic is lignocaine hydrochloride, procaine hydrochloride, prilocaine hydrochloride, chloroprocaine hydrochloride, etidocaine hydrochloride, or mepivacaine hydrochloride.

4. A preparation according to any preceding claim, wherein the concentration of the local anaesthetic is between 0.01% and 1% w/v, inclusive.

5. A preparation according to claim 4, wherein the concentration of the local anaesthetic is between 0.05% and 0.40% w/v, inclusive.

6. A preparation according to any preceding claim, wherein the concentration of the local anaesthetic is between 0.08% and 0.15% w/v, inclusive.

7. A preparation according to any preceding claim, wherein the poloxamer is selected from the group consisting of; P188, P234, P237, P338, and P407.

8. A preparation according to any preceding claim, wherein the Propofol is solubilised in a synergistic mix of poloxamers.

9. A preparation according to claim 8, wherein the poloxamers present in the synergistic mix are selected from the group consisting of; P188, P234, P237, P338, and P407.

10. A preparation according to any preceding claim comprising at least two poloxamers and wherein each poloxamer forms at least 10% of the total poloxamer present in the preparation.

11. A preparation according to claim 10, wherein each poloxamer forms at least 20% of the total poloxamer present in the preparation.

12. A preparation according to claim 10, wherein each poloxamer forms at least 30% of the total poloxamer present in the preparation.

13. A preparation according to any preceding claim, having two poloxamers therein.

14. A preparation according to claim 13, wherein the poloxamers are P407 and P188.

15. A preparation according to claim 13 or 14, wherein the poloxamers are in a ratio to each other of about 7 : 3 by weight.

16. A preparation according to claim 15, wherein the poloxamers are P407 and P188 and are in a ratio to each other of about 7 : 3 by weight, respectively.

17. A preparation according to any preceding claim, wherein the poloxamer forms between 3% and 20% w/v of the preparation.

18. A preparation according to claim 17, wherein the poloxamer forms between 5 and 15% w/v of the preparation.

19. A preparation according to any preceding claim, comprising at least 0.5% w/w Propofol.

20. A preparation according to claim 19, comprising at least 1% w/w Propofol.

21. A unit dose formulation of a preparation according to any preceding claim, comprising a therapeutically effective amount of Propofol.

22. A multiple unit dose formulation according to claim 21.

23. A formulation according to claim 21 or 22, provided in a sealed container.

24. Use of Propofol and a local anaesthetic in the manufacture of an aqueous, micellar poloxamer preparation medicament to reduce localised pain on delivery of said Propofol.

## Patentansprüche

1. Wässriges, mizellares Poloxamer-Präparat, aufweisend Propofol und ein Lokalanästhetikum.

2. Präparat nach Anspruch 1, worin das Anästhetikum ein ionisch dissoziierbares, wasserlösliches Lokalanästhetikum ist.

3. Präparat nach Anspruch 1 oder 2, worin das Anästhetikum Lidocain-hydrochlorid, Procain-hydrochlorid, Pridocain-hydrochlorid, Chloroprocain-hydrochlorid, Etidocainhydrochlorid oder Mepivacain-hydrochlorid ist.

4. Präparat nach einem der vorgenannten Ansprüche, worin die Konzentration des Lokalanästhetikums zwischen 0,01% und 1% (Gewicht/Volumen) einschließlich liegt.

5. Präparat nach Anspruch 4, worin die Konzentration des Lokalanästhetikums zwischen 0,05% und 0,40% (Gewicht/Volumen) einschließlich liegt.

6. Präparat nach einem der vorgenannten Ansprüche, worin die Konzentration des Lokalanästhetikums zwischen 0,08% und 0,15% (Gewicht/Volumen) einschließlich liegt.

7. Präparat nach einem der vorgenannten Ansprüche, worin das Poloxamer ausgewählt ist aus der Gruppe, bestehend aus P188, P234, P237, P338 und P407.

8. Präparat nach einem der vorgenannten Ansprüche, worin das Propofol in einer synergistischen Mischung von Poloxameren solubilisiert ist.

9. Präparat nach Anspruch 8, worin die Poloxamere, die in der synergistischen Mischung vorliegen, ausgewählt sind aus der Gruppe, bestehend aus P188, P234, P237, P338 und P407.

10. Präparat nach einem der vorgenannten Ansprüche, aufweisend mindestens zwei Poloxamere, worin jedes Poloxamer mindestens 10% des in dem Präparat vorhandenen Gesamt-Poloxamers ausmacht.

11. Präparat nach Anspruch 10, worin jedes Poloxamer mindestens 20% des in dem Präparat vorhandenen Gesamt-Poloxamers ausmacht.

12. Poloxamer nach Anspruch 10, worin jedes Poloxamer mindestens 30% des in dem Präparat vorhandenen Gesamt-Poloxamers ausmacht.

13. Präparat nach einem der vorgenannten Ansprüche, das darin zwei Poloxamere aufweist.

14. Präparat nach Anspruch 13, worin die Poloxamere P407 und P 188 sind.

15. Präparat nach Anspruch 13 oder 14, worin die Poloxamere zueinander in einem Gewichtsverhältnis von etwa 7:3 vorliegen.

16. Präparat nach Anspruch 15, worin die Poloxamere P407 und P188 sind und zueinander in einem Gewichtsverhältnis von etwa 7:3 vorliegen.

17. Präparat nach einem der vorgenannten Ansprüche, worin das Poloxamer zwischen 3% und 20% (Gewicht/Volumen) des Präparates ausmacht.

18. Präparat nach Anspruch 17, worin das Poloxamer zwischen 5% und 15% (Gewicht/Volumen) des Präparates ausmacht.

19. Präparat nach einem der vorgenannten Ansprüche, aufweisend mindestens 0,5% (Gewicht/Gewicht) Propofol.

20. Präparat nach Anspruch 19, aufweisend mindestens 1% (Gewicht/Gewicht) Propofol.

21. Zubereitungsform in Einheitsdosis eines Präparates nach einem der vorgenannten Ansprüche, aufweisend eine therapeutisch wirksame Menge von Propofol.

22. Zubereitungsform in Mehrfachdosis nach Anspruch 21.

23. Zubereitungsform nach Anspruch 21 oder 22, bereitgestellt in einem verschlossenen Behälter.

24. Verwendung von Propofol und eines Lokalanästhetikums in der Herstellung eines Medikamentes mit einem wässrigen, mizellaren Poloxamerpräparat zur Verminderung des lokalisierten Schmerzes bei der Zuführung des Propofols.

## Revendications

1. Préparation de poloxamère micellaire, aqueuse comprenant du Propofol et un anesthésique local.

2. Préparation suivant la revendication 1, dans laquelle l'anesthésique est un anesthésique local, soluble dans l'eau, ioniquement dissociable.

3. Préparation suivant la revendication 1 ou 2, dans laquelle l'anesthésique est un chlorhydrate lignocaïne, un chlorhydrate de procaïne, un chlorhydrate de prilocaïne, un chlorhydrate de chloroprocaïne, un chlorhydrate d'étidocaïne ou un chlorhydrate de mépivacaïne.

4. Préparation suivant l'une quelconque des revendications précédentes, dans laquelle la concentration de l'anesthésique local est entre 0,01% et 1% p/v, inclus.

5. Préparation suivant la revendication 4, dans laquelle la concentration de l'anesthésique local est entre 0,05% et 0,40% p/v, inclus.

6. Préparation suivant l'une quelconque des revendications précédentes, dans laquelle la concentration de l'anesthésique local est entre 0,08% et 0,15% p/v, inclus.

7. Préparation suivant l'une quelconque des revendications précédentes, dans laquelle le poloxamère est choisi dans le groupe constitué de: P188, P234, P237, P338 et P407.

8. Préparation suivant l'une quelconque des revendications précédentes, dans laquelle le Propofol est solubilisé dans un mélange synergique de poloxamères.

9. Préparation suivant la revendication 8, dans laquelle les poloxamères présents dans le mélange synergique sont choisis dans le groupe constitué de: P188, P234, P237, P338 et P407.

10. Préparation suivant l'une quelconque des revendications précédentes, comprenant au moins deux poloxamères et dans laquelle chaque poloxamère forme au moins 10% du poloxamère total présent dans la préparation.

11. Préparation suivant la revendication 10, dans laquelle chaque poloxamère forme au moins 20% du poloxamère total présent dans la préparation.

12. Préparation suivant la revendication 10, dans laquelle chaque poloxamère forme au moins 30% du poloxamère total présent dans la préparation.

13. Préparation suivant l'une quelconque des revendications précédentes, possédant deux poloxamères dans celle-ci.

14. Préparation suivant la revendication 13, dans laquelle les poloxamères sont P407 et P188.

15. Préparation suivant la revendication 13 ou 14, dans laquelle les poloxamères sont dans un rapport de l'un sur l'autre d'environ 7:3 en poids.

16. Préparation suivant la revendication 15, dans laquelle les poloxamères sont P407 et P188 et sont dans un rapport de l'un sur l'autre d'environ 7:3 en poids, respectivement.

17. Préparation suivant l'une quelconque des revendications précédentes, dans laquelle le poloxamère forme entre 3% et 20% p/v de la préparation.

18. Préparation suivant la revendication 17, dans laquelle le poloxamère forme entre 5 et 15% p/v de la préparation.

19. Préparation suivant l'une quelconque des revendications précédentes, comprenant au moins 0,5% p/p de Propofol.

20. Préparation suivant la revendication 19, comprenant au moins 1 % p/p de Propofol.

21. Formulation de dose unitaire d'une préparation suivant l'une quelconque des revendications précédentes, comprenant une quantité thérapeutiquement efficace de Propofol.

22. Formulation de doses unitaires multiples suivant la revendication 21.

23. Formulation suivant la revendication 21 ou 22, fournie dans un récipient scellé.

24. Utilisation de Propofol et d'un anesthésique local dans la fabrication d'un médicament de préparation de poloxamère micellaire, aqueuse pour réduire une douleur localisée lors de la libération dudit Propofol.
